# EUROPEAN PATENT SPECIFICATION

(11) **EP 4 142 655 B1**
(45) Date of publication and mention of the grant of the patent: **25.02.2026**
(21) Application number: 21833260.9
(22) Date of filing: 16.06.2021
(51) Int. Cl.: A61F 2/46, A61B 17/56, A61F 2/30, B05B 7/02

(54) **BONE PREPARATION SYSTEM**
KNOCHENPRÄPARATIONSSYSTEM
SYSTÈME DE PRÉPARATION OSSEUSE

(30) Priority: 30.06.2020 US 202063046357 P; 10.06.2021 US 202117343989
(43) Date of publication of application: 08.03.2023
(73) Proprietor: Kinamed, Inc., Camarillo, CA 93012-8701 (US)
(72) Inventor: NELSON, Adam Lee, Ventura, California 93003 (US); SARIN, Vineet Kumar, Moorpark, California 93021 (US); BRUCE, Robert Arthur, Ventura, California 93003 (US); MENEGHINI, Robert Michael, Fishers, Indiana 46037 (US); PRATT, William Ralph, Newbury, California 91320 (US)
(74) Representative: South, Nicholas Geoffrey
(86) International application number: PCT/US2021/037658
(87) International publication number: WO 2022/005752

(56) References cited:
- WO-A1-99/12595
- CN-U- 204 307 896
- DE-A1- 3 108 918
- US-A- 4 583 531
- US-A- 4 627 434
- US-A- 5 520 667
- US-A- 5 704 909
- US-A1- 2006 142 690
- US-A1- 2014 364 817

## Description

### BACKGROUND

In traditional joint replacement surgery, bone is prepared to receive a prosthetic implant by first cutting or sculpting the bone with a manual or powered tool such as a saw, drill, or broach which generally exposes the more porous inner cancellous bone. Next, the exposed bone surface is usually cleaned with a sterile saline solution for removal of bone, blood and other tissue debris, and suction is applied to remove the saline fluid and debris. Often, surgical sponges are inserted into a cavity or against the bone surface to absorb excess fluids.

Joint replacements are commonly but not necessarily secured with the aid of "bone cement," a biocompatible grouting material; a typical bone cement is polymethylmethacrylate (PMMA). The success of such bone cements is thought to depend in part on proper preparation of not only the bone surface but especially the deeper cancellous "bone bed".

U.S. Pat. No. 5,037,437 to Matsen Ill (1991) discloses a significant improvement in the art of preparing bone surface and the deeper bone bed for cemented joint replacement surgery. Matsen identified some of the previously unrecognized shortcomings of traditional liquid flushing lavage for preparation of the cancellous portions of an exposed bone bed. Matsen's invention was based on the finding that dry flowing gas directed at and into the sculpted bony bed effectively prepares the bone for cemented prosthetic implantation by cleaning debris more thoroughly and from deeper in the bone bed. When bone cement is used, the use of gas increases the likelihood of strong mechanical interdigitation of the bone cement with the bone. Matsen also suggests that carbon dioxide gas (CO₂) is especially well suited for use as the dry gas for bone preparation, having already been demonstrated safe for use in the human body. Carbon dioxide gas is commonly available in hospital operating rooms, finding decades of use in laparoscopic surgery, for example.

Since the publication of the Matsen patent, tools have become available for preparing bony surfaces using sterile, dry gas or sterile admixtures of gas and liquid. A CO₂ bone preparation system is available, for example, from Kinamed, Inc. in Camarillo, Calif (marketed under the trade name "CarboJet"). However, certain essential components of this system must be sterilized prior to each use. The sterilization procedure is typically performed at a location remote from the operating room, which is necessarily costly and time-consuming.
DE 3108918 A1 discloses a device for rinsing, cooling and spraying body cavities, especially bone bearings in which endoprostheses are fixed with bone cement. The device comprises a pressurised gas container, a metering device, storage vessels for liquids or powders and specially shaped dispensing attachments which are inserted into the body cavity or the bone bearing. Compressed gases are used as pressurising agents and coolants.
US 2014/364817 A1 discloses a medical spraying device and for irrigating a wound, including a liquid reservoir for a medical irrigation liquid or a connection for such a liquid reservoir, and a compressed gas reservoir. The compressed gas reservoir is connected via a pressure line to the liquid reservoir, such that the liquid can be pushed by the gas pressure of the compressed gas reservoir through a nozzle to produce a spray cone.
CN 204307896 U discloses a minimally invasive surgery endoscope cleaning gun capable of adjusting flow, and comprising a flow adjusting button, a hook, a gun body, a conical flushing nozzle, a trigger control water valve, a water valve outer cap, a spring, a water liquid flow channel, a water tube connector, a handle, a water valve element water through hole, a water valve element channel and a flow control cone head.
WO 99/12595 A1 discloses a disposable atomizer device including a container for liquid, a liquid inlet tube, an atomizing nozzle, a gas inlet tube, a trigger valve system and a needle valve.

### SUMMARY

A disposable CO₂ bone preparation system is described. Certain essential system components (including tube set, hand piece, and nozzle) that are necessary to perform dry gas bone preparation are provided in a single package. No assembly or sterilization of these components need be performed by the user; the system is ready to use once the package is opened and the tube set is coupled to a regulated, medical grade CO₂ source.
In one aspect, the present invention provides a bone preparation system as defined in claim 1. In another aspect, the present invention provides a bone preparation system kit as defined in claim 7.

Features of the present system include:
- Fully disposable delivery system that attaches to a regulated, medical grade CO₂ source
- No assembly or sterilization required by user; the system is ready to use once opened
- Nozzle can be rotated to allow for helpful angles of attack, especially for preparation in the back of a joint
- Optional (snap-on and removable) splash shield reduces debris spray caused by application of pressurized gas

### DETAILED DESCRIPTION OF THE DRAWINGS

FIG. 1 is a perspective view of a bone preparation system kit;
FIG. 2 is an enlarged perspective view of the hand piece and nozzle of the bone preparation system of FIG. 1;
FIG. 3 is an enlarged side elevation view of the hand piece and nozzle of FIG. 2;
FIG. 4 is a perspective view of the nozzle with an optional shield mounted to the nozzle;
FIG. 5A is a bottom plan view of the nozzle and optional shield of FIG. 4;
FIG. 5B is another perspective view the nozzle and optional shield of FIG. 4;
FIG. 6 is a perspective view of the nozzle with another optional shield mounted to the nozzle;
FIG. 6A is an enlarged bottom plan view of the tip of the nozzle and an optional shield mounted to the nozzle of FIG. 6;
FIG. 7A is a side elevation view of the hand piece of FIG. 1 with another optional shield mounted to the nozzle;
FIG. 7B is an enlarged perspective view of the optional shield of FIG. 7A;
FIG. 7C is a side elevation view of yet another optional shield;
FIG. 7D is a perspective view of the shield of FIG. 7C;
FIG. 8 is a side elevation view of another embodiment of a hand piece;
FIG. 9 is a side elevation view of another embodiment of a bone preparation system kit with a third embodiment of a hand piece, with ergonomic packaging;
FIG. 10 is an enlarged side elevation of the hand piece of FIG. 9 with another embodiment of an optional shield;
FIG. 11 is an enlarged side elevation of the hand piece nozzle tip and shield of FIG. 10;
FIG. 12 is an enlarged exploded perspective view of the nozzle with the shield removed;
FIG. 13 is another enlarged view of the nozzle tip and optional shield;
FIG. 14 is cross-section view taken along line XIV-XIV of FIG. 10 rotated 180 degrees;
FIG. 14A is an enlarged cross-section view of the mounting connection of the nozzle to the hand piece body;
FIG. 15 is a perspective view of the bone preparation system held by a user with another tube shown supported by the hand piece for applying suction or for delivering light to the preparation site;
FIG. 16 is an exploded partial fraamentarv perspective view of a nozzle tip suitable for use with a trunnion;
FIG. 17A is an exploded partial fragmentary perspective view of a nozzle tip suitable for use with a trunnion;
FIG. 17B is a cross-section of a nozzle tip suitable for use with a trunnion;
FIG. 17C is a cross-section of a nozzle tip suitable for use with a trunnion;
FIG. 17D is a similar view to FIG. 17C illustrating the flow of gas through the nozzle tip;
FIG. 17E is an enlarged cross-section of the lower end of the body illustrating the annular passageway that forms an air knife;
FIG. 17F is a similar view to FIG. 17C illustrating the flow of gas to the outlet of the nozzle tip; and
FIG. 18 is a perspective view of a trunnion collar.

### DETAILED DESCRIPTION

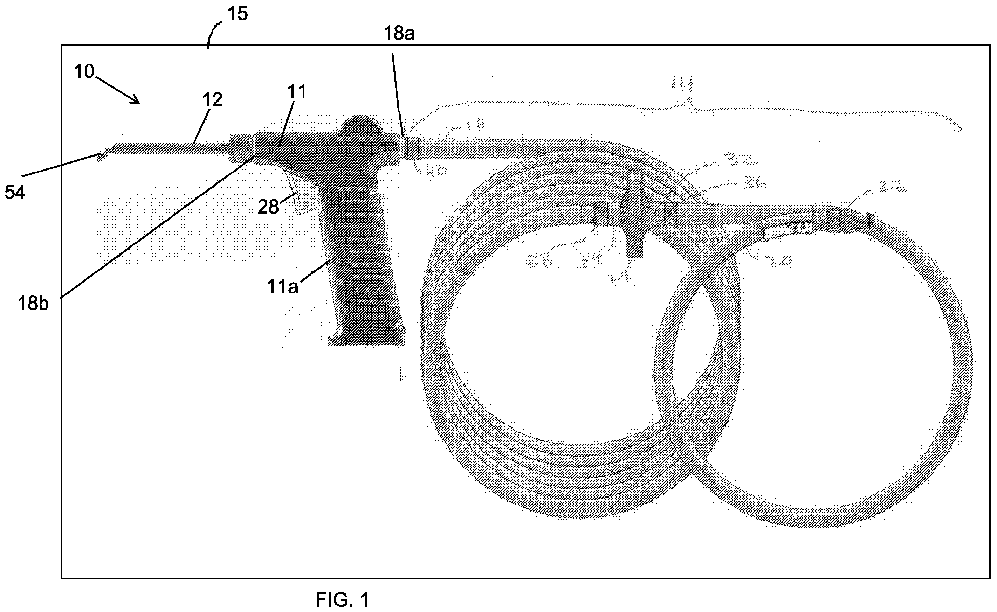

Referring to FIG. 1, the numeral 10 generally designates a bone preparation system. As will be more fully described below, bone preparation system 10 is configured so that it can pre-sterilized and ready for use and further packaged in a sealed package 15, described below, to keep the system sterile. Additionally, bone preparation system 10 may be disposable.

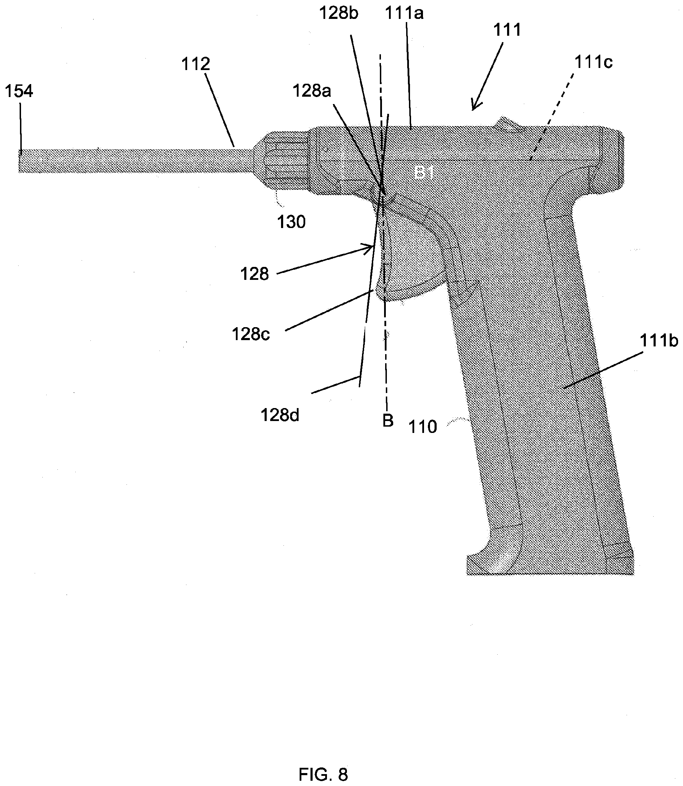

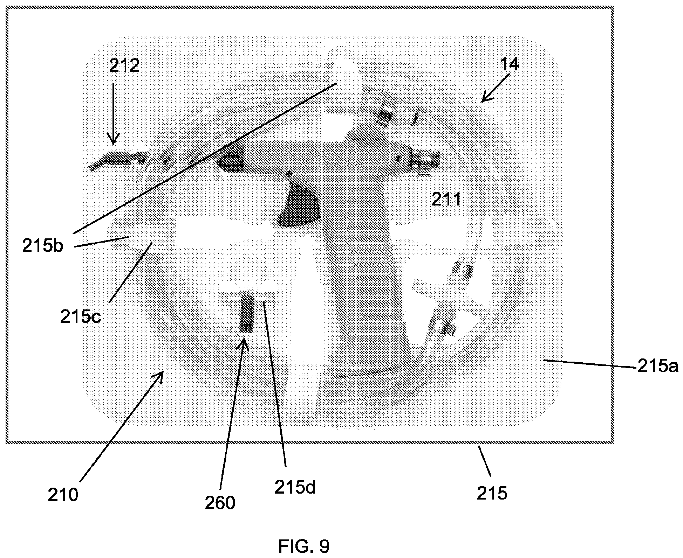

As best seen in FIG. 1, in the illustrated embodiment, bone preparation system 10 includes a hand piece 11, a nozzle 12, and a tube set 14 (additional views of other embodiments of a hand piece and nozzle are shown in FIGS. 8 and 9), which is configured so that it be connected to a CO₂ source for bone preparation. Each system component is pre sterilized and provided together in a sealed package 15, such as a pouch. Package 15 may be formed from a flexible, and optionally frangible material, such as plastic, including Tyvek or mylar, or metal foil or the like or a combination thereof, which may include a seam so the package can be manually peeled opened at the seam. For example, package 15 may formed as a sealed pouch, such as a sealed plastic (e.g. Tyvek or mylar) pouch or a metal foil pouch. Alternately, the package may be formed by a tray, such as a tray made out of polyethylene terephthalate glycol (PETG), with a sealed lid, such as a Tyvek. Optionally, each bone preparation system is sealed within its packaging and is pre-sterilized, such as by subjecting the packaged and sealed bone preparation system to a gamma sterilization process. As will be more fully described below, each bone preparation system may be configured as a single-use device, after which it is disposed of.

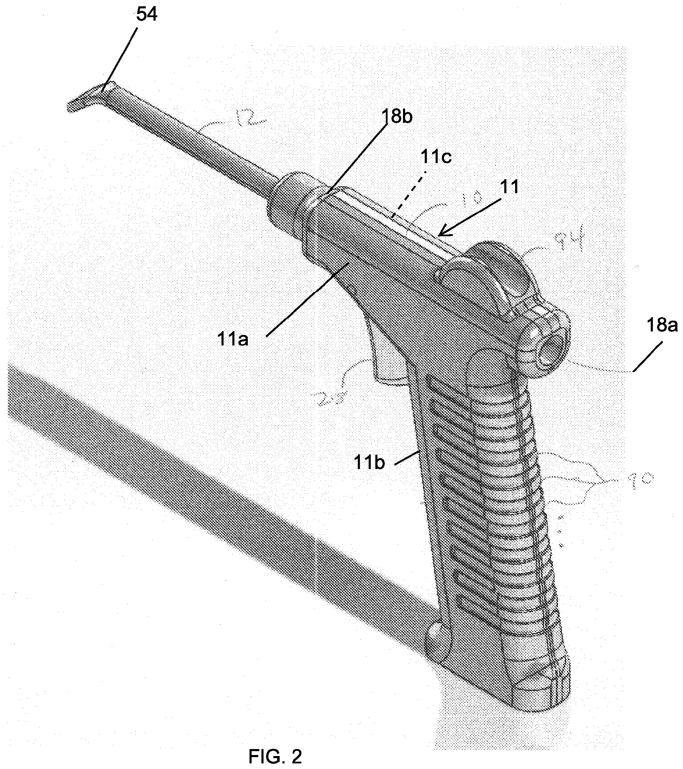

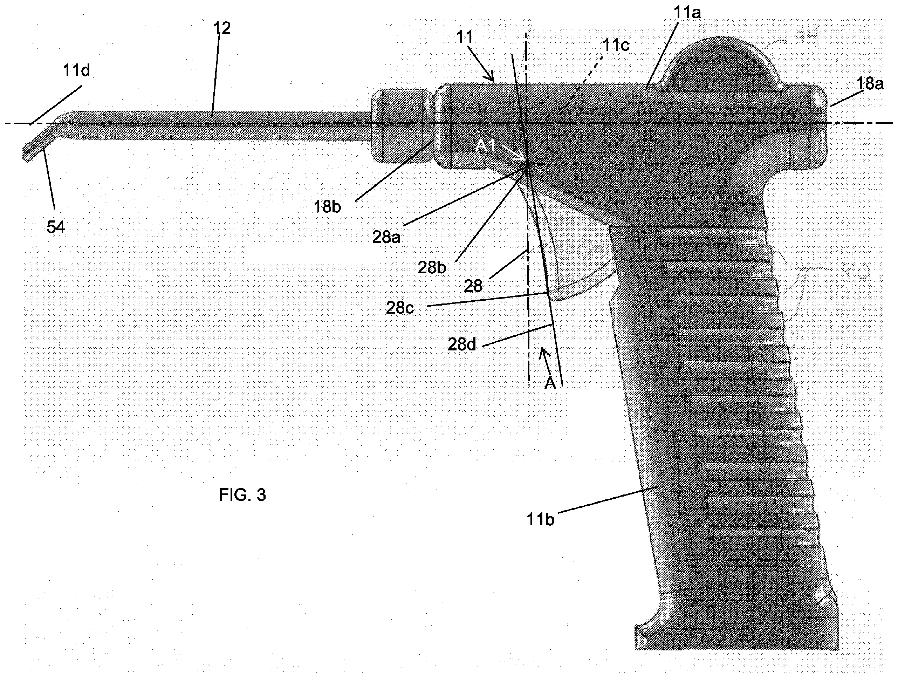

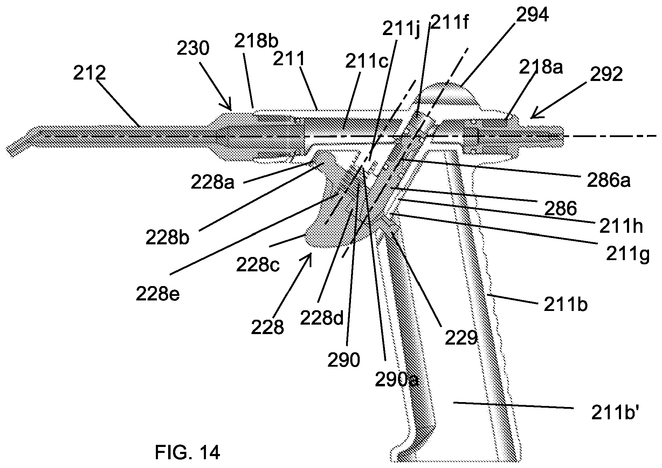

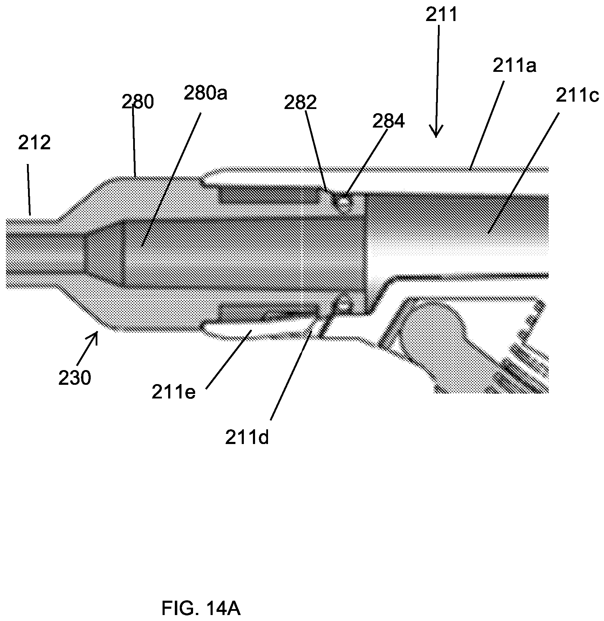

As best seen in FIG. 2, hand piece 11 includes body 11a, which forms a hand grip 11b and an internal passage 11c that extends between and is in fluid communication with an inlet port 18a and an outlet port 18b to which nozzle 12 is mounted and through which the CO₂ gas can flow. As will more fully described below in reference to another embodiment and shown FIG 14, internal passageway 11c forms a straight or linear flow path along a longitudinal axis 11d (FIG. 3) that extends through hand piece body 11a, which provides for low frictional and energy losses that may result in a relatively smooth laminar flow of gas from outlet port 18b. The gas flow through internal passageway 11c is modulated by a trigger 28, described more fully below.

Body 11a may be formed from plastic, such as thermoplastic polymer, as noted below, and is molded into its desired shaped, optionally, in two halves, which are then joined together by welding or fasteners, such as rivets or screws, or may be bonded together such as with an adhesive. Nozzle 12 may too be formed from plastic or metal.

Trigger 28 is a wedged-shaped member (also optionally molded from plastic) that is pivotally mounted at one corner thereof to body 11a by a pivot member 28a (FIG. 3) about a pivot axis 28b (FIG. 3). In this manner, trigger 28 can pivot between a fully compressed position in which the trigger 28 is fully pivoted into the body 11a, which corresponds to the internal passageway being fully opened, and an uncompressed position in which the internal passageway is closed - and optionally any position in between to modulate the flow of gas through the internal passageway. Optionally, trigger 28 may be mounted to provide an open or closed position only, though greater control is provided over a trigger than can be moved to any position in between. For further optional details of how trigger 28 may be mounted, reference is made to the description and illustration in FIG. 14.

In the illustrated embodiment, as best seen in FIG. 3, trigger 28 is mounted so that in its uncompressed position its distal tip 28c is tangent to line 28d that extends through pivot axis 28b. Line 28d forms an acute angle A in the clockwise direction relative to a vertical axis passing through pivot axis 28b (when the hand piece 11 is oriented with its internal passageway 11c aligned along the horizontal axis). Or stated another way, line 28d forms an obtuse angle A1 in the clockwise direction relative to the longitudinal axis of internal passage way 11c.

Optionally, as best seen in FIG. 1, end 16 of the tube set 14 may be permanently attached to port 18a at the rear of hand piece 11 with a snap fit fitting 40, which when inserted cannot be removed, with the other end 20 equipped with a fitting 22 for connection to a regulated, medical grade CO₂ source that is typically located outside of the sterile field. When so arranged, there is no assembly required-once removed from package 15 and once the fitting 22 is connected to a CO₂ source, the system is ready to use. The tube set may also include an inline filter 24 that serves to clean and ideally ensure sterile delivery of the medical grade CO₂ gas.

Alternately, tube set 14 may connect to hand piece 11 at port 18a via a bayonette style fitting (such as shown in reference to hand piece 211 in FIG. 14), which may allow removal of the tube set.

In use, a user grips the hand piece 11, places the tip of the nozzle 12 near an area of bone to be prepared, and compresses trigger 28 to start the flow of gas, which also as noted may allow modulation of the flow of gas between a non-flow state and a full flow state. Additional control over the direction of the gas flow is preferably provided by making nozzle 12 rotatable around its longitudinal axis, which may help achieve a better angle with which to clean a particular bone surface, and/or by the style of the nozzle tip 54.

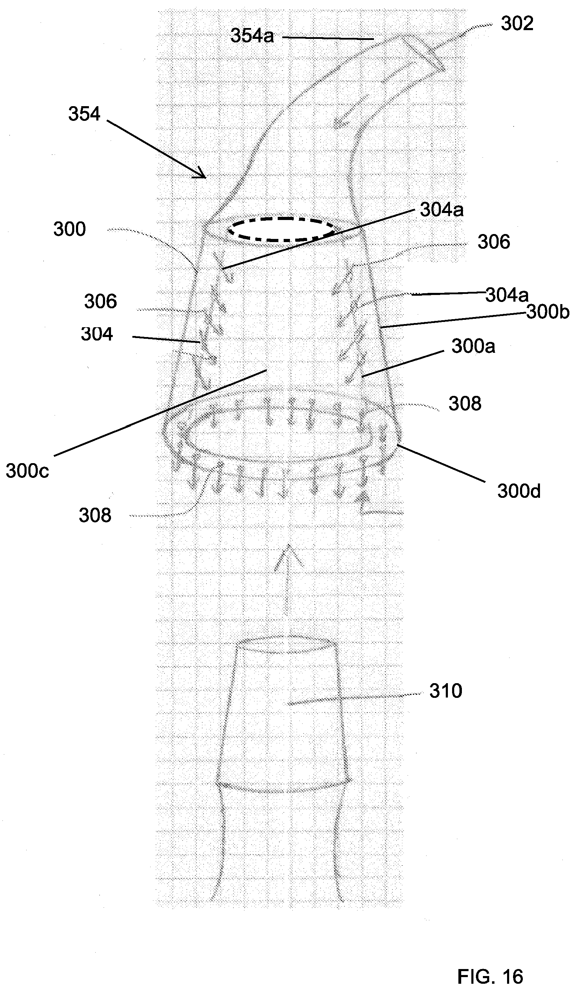

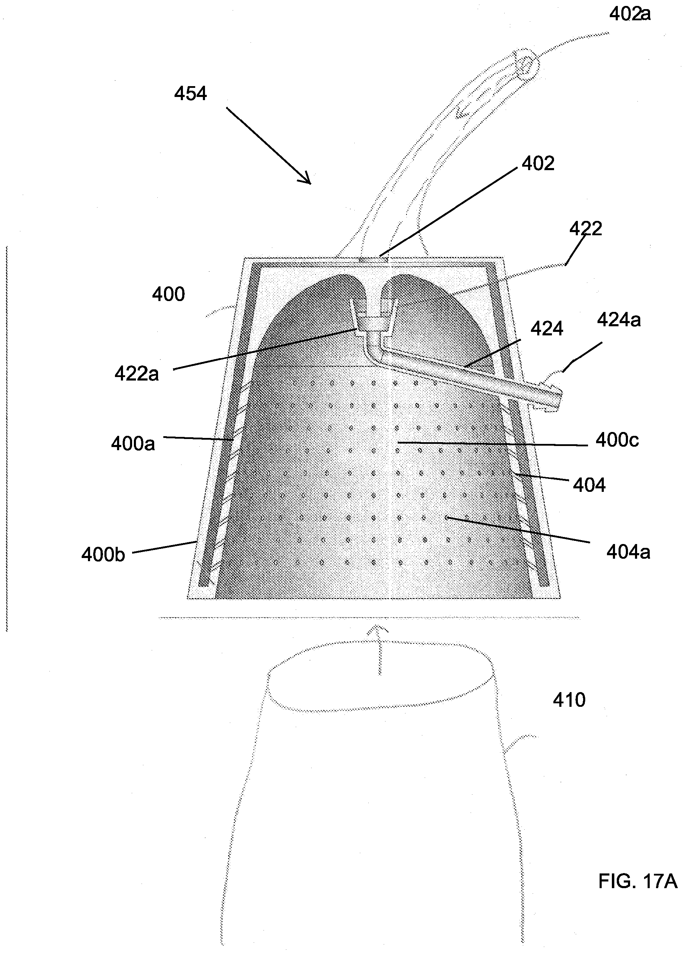

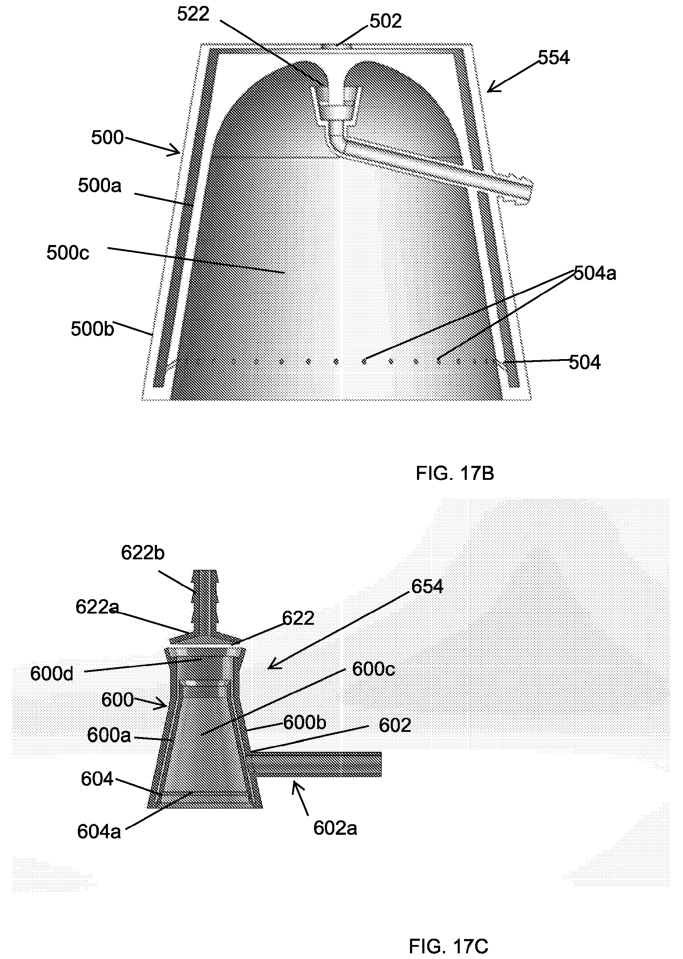

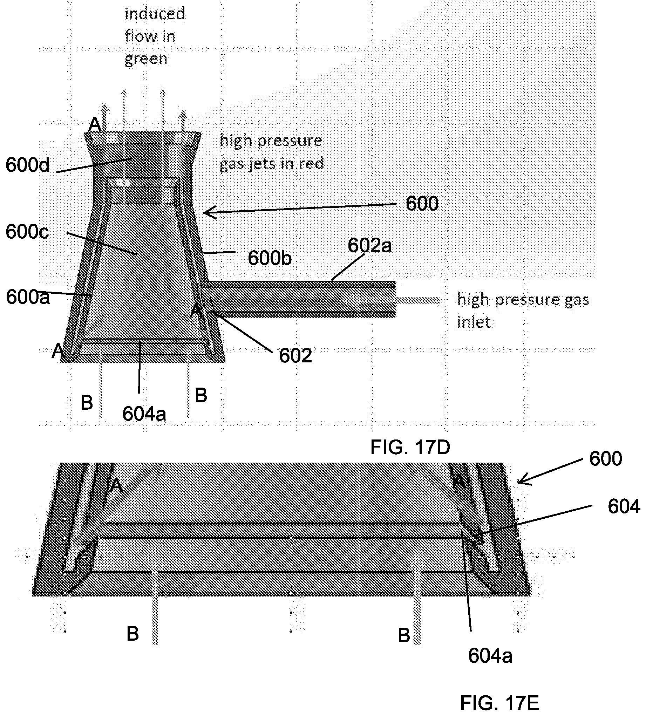

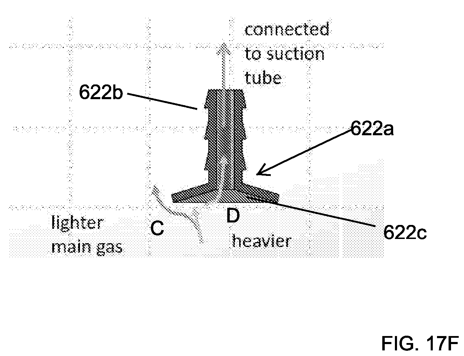

In the illustrated embodiment of FIG. 1, nozzle tip 54 is configured for treatment of knee replacement, while nozzle tip 154 in FIG. 8 may is more suitable for long bone preparation such as with hip or shoulder replacement. As will be more fully described below in reference with FIGS. 16 and 17, other nozzle tip configurations may be used.

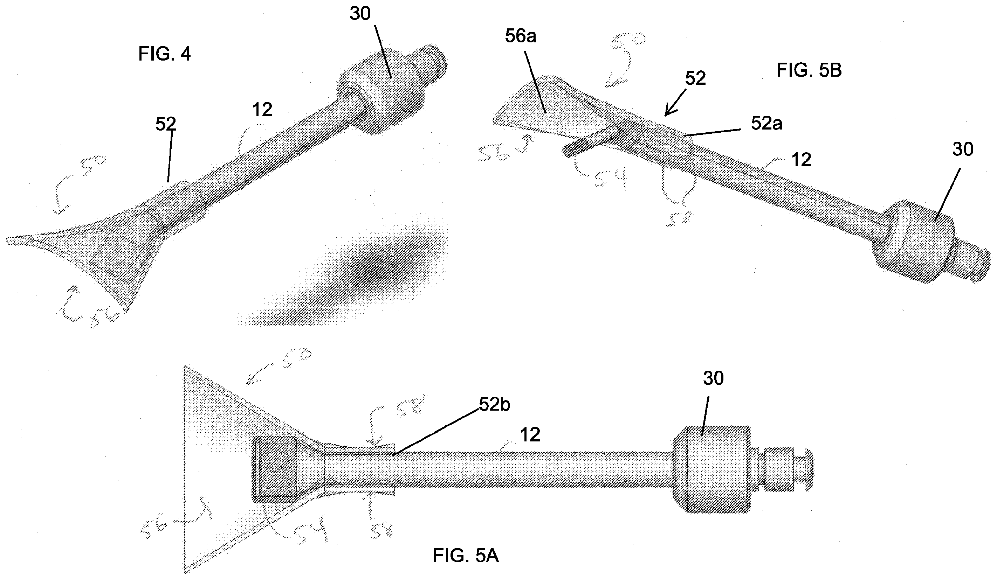

To facilitate rotation of nozzle 12 around its longitudinal axis, nozzle 12 may include an enlarged collar 30 in FIG. 4 or 130 in FIG. 8 at its connection to hand piece 11 that can be gripped by a user's finger and thumb that make it easier to rotate the nozzle. Further, as shown in reference to hand pieces 111 and 211 (FIGS. 8 and 9), the collar may incorporate recesses to provide a tactile feedback feature. Though nozzle 12 may be rotatable, it would typically not be removable from hand piece 11. Again, this allows for a simpler construction and encourages one time use of the hand piece.

As noted above, hand piece 11 and nozzle 12 (and trigger 28) may be made from a plastic, such as thermoplastic polymer, such as ABS. The use of a plastic for the hand piece and nozzle is advantageous, as the material's low thermal conductivity helps insulate the user's hand from the low temperature of the flowing, pressurized CO₂ gas, and its light weight helps reduce user fatigue that might otherwise occur. Further, as noted above, the hand piece may be formed from two pieces, which are joined together, and further may be formed from two hollow pieces, with the internal passageway and other mounting structures discussed below being formed by internal ribs and walls so that the weight of the hand piece can be further reduced.

The sterilization process, for example, a gamma sterilization process, tends to degrade certain plastics. While some plastics are "gamma stable" and degrade so slowly as to not be noticeable, others degrade faster. As such, the rate at which the selected plastic degrades will affect the system's functional shelf life.

The tubing portion included as tube set 14 is also formed from a plastic, and typically a more flexible plastic, such as polyvinyl chloride (PVC). Referring back to FIG. 1, the tube set's inline filter 24 includes an inlet 32 and an outlet 34, and is optionally gamma stable, HEPA-rated, and capable of maintaining high flow rates. The tube set would typically include four fittings: Fitting 22 for connection to a regulated medical grade CO₂ source, fittings 36 and 38 for connection to the inlet 32 and outlet 34 of filter 24, respectively, and fitting 40 for connection to port 18 on the rear of hand piece 11. Fittings 36 and 38 may be identical and optionally integrated into the filter, while fittings 22 and 40 may have different configurations to provide the appropriate connections with the regulator and the hand piece port 18b, respectively.

The materials with which the fittings are made can also affect the system's functional shelf life, as they will also be subjected to the sterilization process. Suitable fitting types includes barbed with crimp, barbed with ferrule, and pocket with glue, with barbed with ferrule typically preferred. It would also be possible to use only barbed connections, though this may require an alternate type of tubing.

An optional feature that may be provided as part of the present disposable CO₂ bone cleaning system or as a stand-alone device for use with reusable bone cleaning systems is a shield, which can be configured to prevent the splash or spray of fluid and debris, and may be configured to snap onto the system's nozzle, which are more fully described below.

Referring back to FIGS 2 and 3, the present system also optionally includes horizontal ribs 90 on the hand grip 11b of hand piece 11, to provide a non-slip grip for a user. These ribs may be formed during molding of hand piece body 11 and/or may be formed or enhanced by over molding or co-injection molding of a resilient or elastomeric material, such as rubber, to increase the friction between a user's hand and the hand piece.

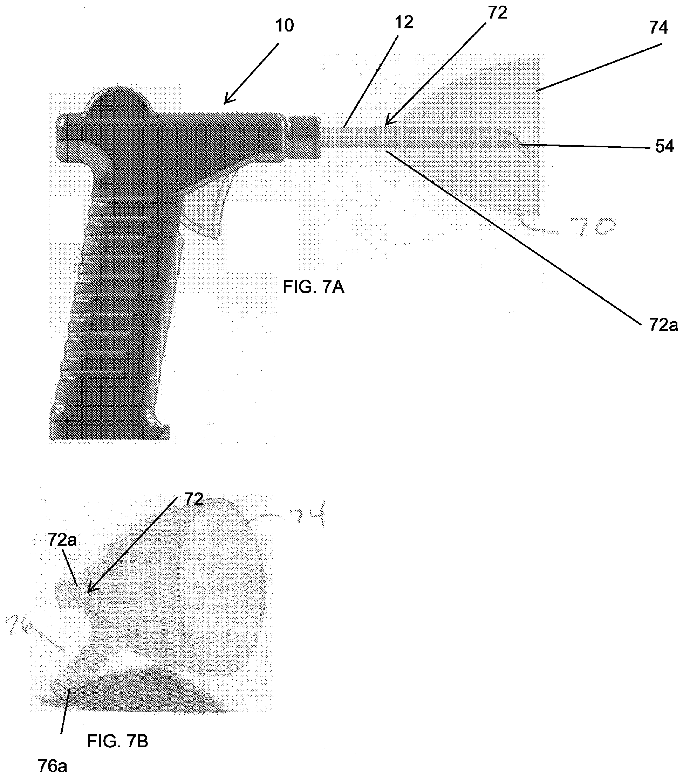

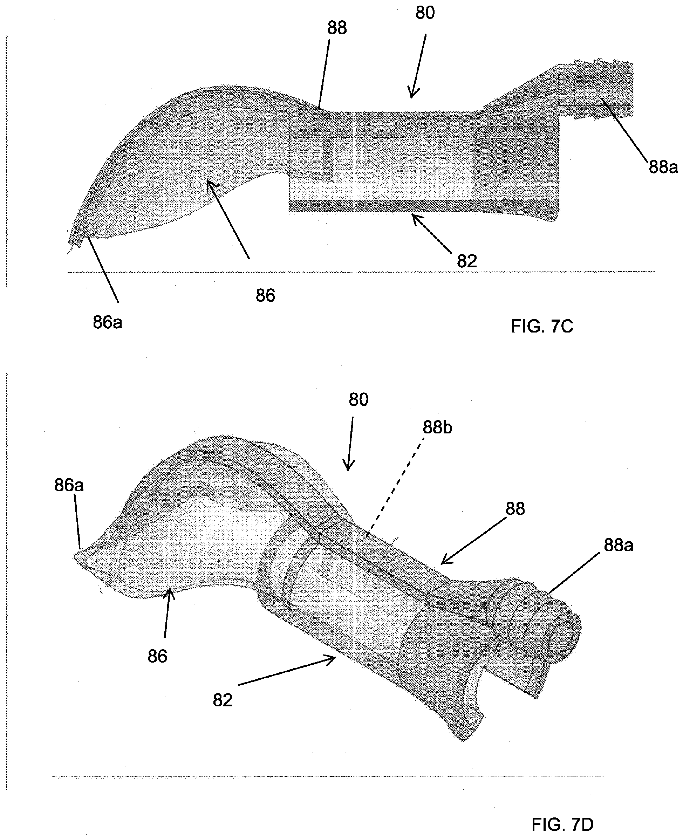

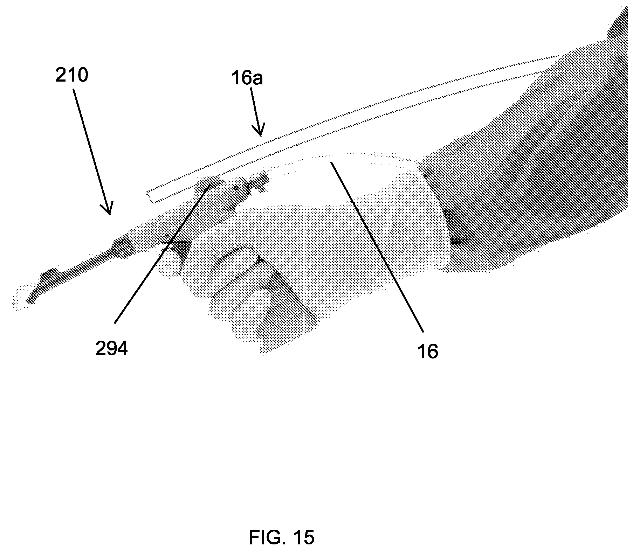

The hand piece may also include a U-shaped channel 94 on the topside of hand piece 11 above grip 11b, which is configured to provide a frictional grip of tubing inserted therein, which may be used, for example, to provide suction as discussed in connection with FIGS. 7B-7D below or to grip a tube in the form of a "light pipe" that projects light into or onto the bone being prepared (see FIG. 15 and corresponding description). Channel 94 may be formed when molding body 11a. Again, ribs and/or a resilient or elastomeric material may be molded on the inside of channel 94 to increase the friction forces on tubing inserted therein.

Referring to FIGS. 4, 5A and 5B, the numeral 50 generally designates one example of a shield. When installed, the shield can reduce the splash of fluid and debris created by the gas flow to the region below the shield. The shield 50 may be made from semi-rigid material, such as plastic or metal, that snaps onto the system's nozzle 12 and maintains its shape under normal conditions, but has the ability to deform when pressed against a rigid or semi-rigid object, allowing the nozzle tip 54 to access and prepare tight spaces, such as the back of a resected proximal tibia surface or a resected posterior femoral condyle. Further, the shield may be made from transparent material, such as a transparent plastic, so that a user can still see the bone which they are preparing.

As best seen in FIGS. 5A and 5B, shield 50 includes a base 52 that forms a collar 52a for mounting the shield to the nozzle adjacent nozzle tip 54. Collar 52a is bifurcated by a split 52b, which forms two spaced terminal edges 58 of collar 52a. Thus, collar 52a forms a curved or C-shaped plate spring with an open side that is smaller than the diameter of the nozzle so that it can be spread and snapped onto nozzle 12, which allows for easy mounting or removal when additional visibility is preferred or after gross removal of fluids or lipids is completed. Edges 58 may be tapered on one or both sides to form a cam surface or cam surfaces to facilitate spreading of the collar when pressed again the nozzle.

Shield 50 also includes a plate 56 that extends from base 52, which is curved or non-planar so that it straddles nozzle tip 54 and forms deflecting surfaces on either side of nozzle tip 54. The opposed edges of plate 56 are continuous with the opposed edges 58 of collar 52a to better wrap around nozzle tip 54, but is generally triangular in shape as seen in FIG 5 so that its distal end is wider than its proximal end adjacent collar 52a, though it may vary, as noted below.

The splash shield is optionally configured so that it is retractable along nozzle 12, such that it is capable of being slid up the longitudinal axis of the nozzle toward the hand piece. In this manner, when the shield is either not needed or when additional visibility or physical access to a particular area is desired, a user may simply move the shield up along the nozzle's longitudinal axis rather than remove it. This can be facilitated through the selection of the material, e.g. low friction material to allow the shield to slide and/or by the spring force exerted by the shield on the nozzle, which is designed to provide the snap fit coupling but a coupling that is loose enough to allow the shield to slide. Optionally, as described in reference to hand piece 211, additional structures may be formed on the nozzle to prevent it from sliding too far or to define a discrete position or discrete retracted positions for the shield along the nozzle. The splash shield can have any of a number of shapes-those illustrated herein are merely examples.

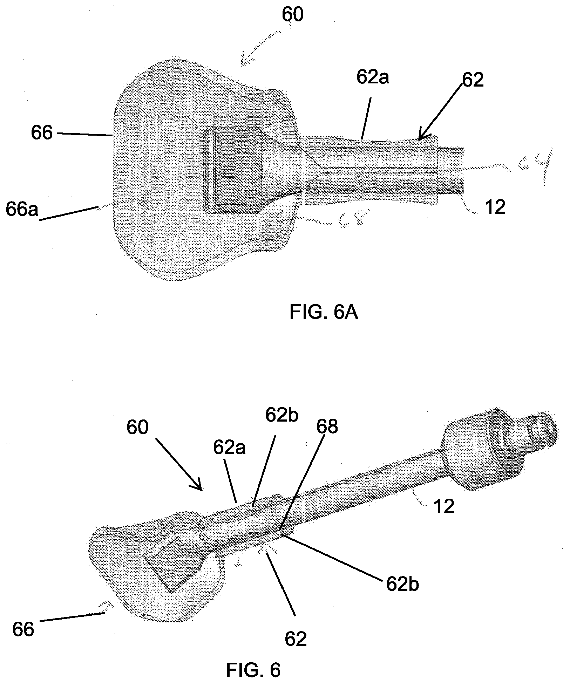

Referring to FIG. 6, the numeral 60 generally designates another example of a shield. Similar to shield 50, shield 60 includes a base 62 that forms a collar 62a for mounting the shield to the nozzle adjacent nozzle tip 54. Collar 62a is also bifurcated by a split 62b, which forms two spaced terminal edges 68 of collar 62a. Thus, collar 62a also forms a curved plate spring with an open side that is smaller than the diameter of the nozzle so that it can be spread and snapped onto nozzle 12.

In the illustrated example, rather than having a straight cylinder collar, like collar 52a, collar 62a may have a curved outer surface or recesses 62b to provide a tactile surface for easier handling of shield 60. Edges 68 may also be tapered on one or both sides to form a cam surface or cam surfaces to facilitate spreading of the collar when pressed again the nozzle.

Shield 60 also includes a plate 66 that extends from base 62, which is also curved or non-planar so that it straddles nozzle tip 54 and forms deflecting surfaces on either side of nozzle tip 54. Plate 66 may have a more curved configuration with a fluted portion of region on the opposed sides plate, which are on either side of nozzle tip 54. Further, they may be located at the transition with collar 62a, and only partially extend around the collar to have a more open design to increase user visibility of the nozzle tip. In other words, the opposed edges of plate 66 are not contiguous with the opposed edges 68 of collar 62a and instead are offset around collar 62a from edges 68.

Referring to FIGS. 7A and 7B, the numeral 70 designates another example of a shield in the form of a cone-shaped shield. Shield 70 provides more expansive shielding than the designs shown in FIGS. 5 and 6, but potentially reduces user visibility. Shield 70 is preferably flexible, clear, and removable, and includes a base 72 forming a collar 72a, which may also use the "split" feature as described above, for mounting shield 70 to nozzle 12 adjacent nozzle tip 54 and a cone-shaped cup 74 that extends from base 72, which fully surrounds nozzle tip 54.

Shield 70 may be configured to provide suction at the distal end of the nozzle, to capture and remove fluids and debris dislodged by the flowing gas. Referring to FIG. 7B, shield 70 may include a suction port 76 in cone-shaped cup 74 that is in fluid communication with the region or space formed around nozzle tip 54. Port 76 may be formed by a generally cylindrical shaped tube, which extends from and may be formed with cup 74, with a cannulated barb 76a to which suction tubing may be attached to form a suction line.

In another example (FIGS. 7C-7D), shield 80, which is similar to shield 60, includes a channel 88, which is formed or provided on the upper surface of shield 80, and runs from the proximal end of base 82 to the distal end of plate 86. For optional details of base 82 and plate 86 reference is made to shield 60.

Channel 88 includes a port 88a in the form of a cylindrical tube that is in fluid communication with internal passageway 88b of channel 88, which may include a cannulated barb 88c for connection with suction tubing to also form a suction line. Here, the vacuum pressure from the suction line is directed by channel 88 to the distal end 86a of the splash shield 80 or plate 86 to effectively remove fluids contained by the shield. While illustrated as provided on the outer surface of shield 80, channel 88 may be internally formed in plate 86 and base 82 so that it maintains the same outer surface topography as shield 60, or it may be formed on in the inside surface of base 82 and/or plate 86.

Referring to FIG. 8, the numeral 111 designates another embodiment of a hand piece that is suitable for use in the system described above. Hand piece 111 is similar to hand piece 11 and includes a hand piece body 111a with an internal passageway 111c and a nozzle 112 mounted to the hand piece body 111a. In the illustrated embodiment, hand piece 111 includes a trigger 128 with a greater range of motion. As best seen in FIG. 8, trigger 128 preferably extends further out from hand piece 111 than it does in the previous embodiment, and hence has a correspondingly longer travel. The hand piece 111 is also optionally designed such that the volume of gas that flows through the nozzle in a given period of time varies with trigger position. As such, the present system's longer travel provides an even greater control over modulation of the gas flow.

In the illustrated embodiment, as best seen in FIG. 8, trigger 128 is mounted so that in its uncompressed position its distal tip 128c extends to the tangent line 128b that extends through pivot axis 128a. Line 128b forms an acute angle B in the counter clockwise direction relative to a vertical axis passing through pivot axis 128a (when the hand piece is oriented with its internal passageway aligned along the horizontal axis). Alternatively, stated another way, line 128b forms an acute angle B1 in the clockwise direction relative to the longitudinal axis of internal passageway 111c.

Further, nozzle 112 includes an adjustment collar 130 that includes a plurality of recesses 130a to form a tactile feedback feature that allows a user to rotate the nozzle as desired about its longitudinal axis.

In the illustrated embodiment, nozzle 112 includes a nozzle tip 154 is configured for long bone preparation and is formed by the terminal end (cut at ninety degrees to the longitudinal axis of nozzle 112). Although not formed with ribs or indentations, handgrip 111b may be formed from a high friction material to improve the grip of a user.

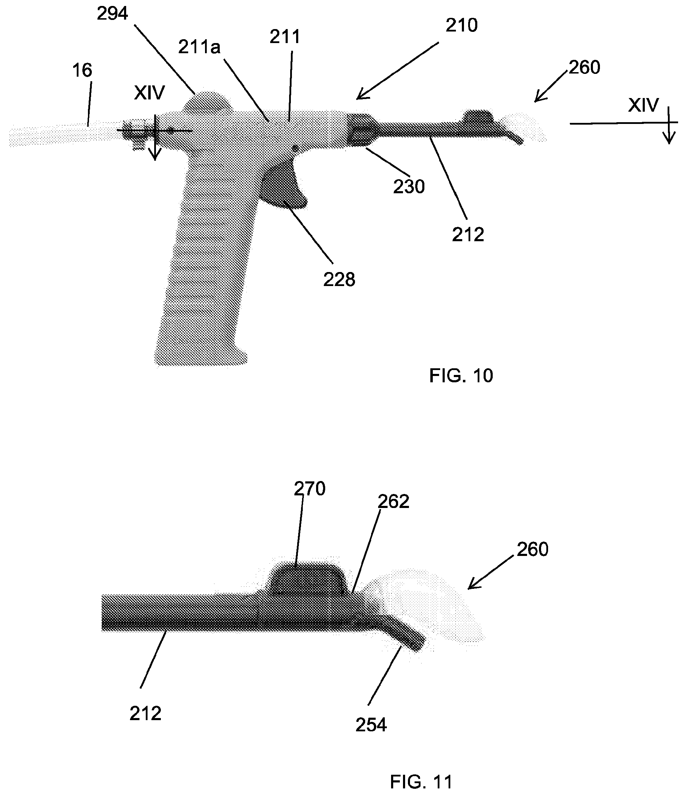

In another embodiment, hand piece 211 (FIGS. 9-11), which is of similar construction to hand pieces 11 and 111, includes a hand piece body 211a, similar to body 111a, a trigger 228, similar to trigger 128, and a modified nozzle 212 with a modified optional shield 260.

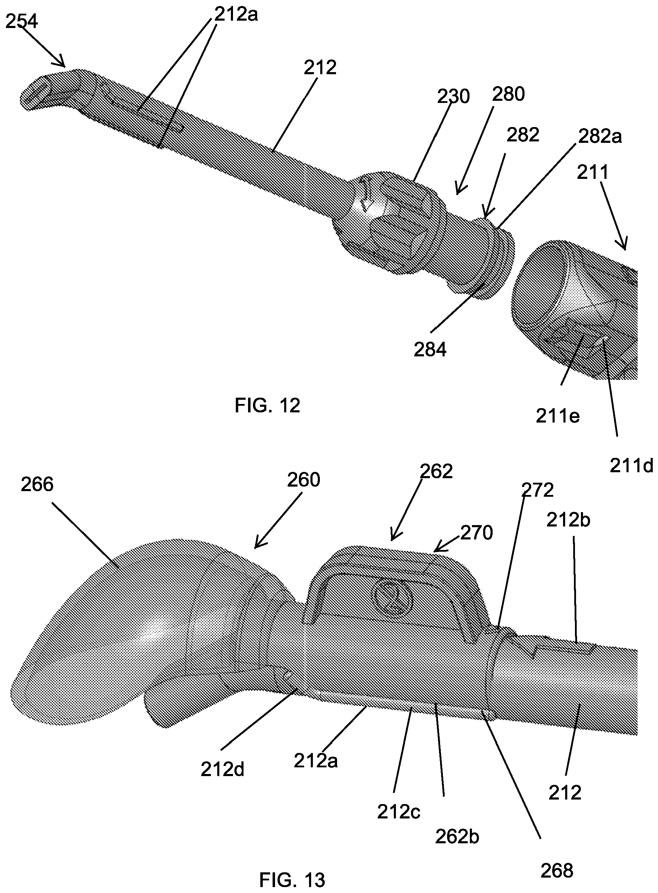

Referring to FIGS. 12-13, nozzle 212 includes a pair of ribs 212a and a stop 212b, which define the proper position of shield 260. Ribs 212a may be formed when molding nozzle 212 and are radially spaced around nozzle 212 adjacent nozzle tip 254 (which is similar to nozzle tip 54). Each rib 212a includes a linear portion 212c, which runs parallel to the longitudinal axis of nozzle 212, and an angle portion 212d that is angled toward the upper side of nozzle 212 to form a guide surface, as well as a stop, for shield 260. Stop 212b is aligned at the proximal end of linear portions 212c and is positioned in the upper side of nozzle 212 to contact and, hence, form a stop for shield 260 when shield 260 is mounted onto nozzle 212. Together angled portions 212d and stop 212b may form a defined position for the shield and further help form the snap fit or friction fit for the shield 260 to reduce unintentional disengagement of the shield from nozzle 212.

In the illustrated example, shield 260 is of similar constriction to shield 60 and includes a base 262 and a curved extended plate 266 that extends from base 262. Base 262 also is formed by a bifurcated collar 262a whose slit 262b is sized so that its opposed edges 268 are spaced so that they must be separated to allow shield 260 to be mounted on nozzle 212. When mounted, opposed edges 268 rest on ribs 212a. In the illustrated embodiment, collar 262a includes a notch on its opposed sides, which matches the angled portions 212d of ribs 212a so that when mounted to nozzle 212, base 262 will either form a snap coupling with nozzle 212 and/or fit tightly in the space defined by the ribs and stop on nozzle 212.

To facilitate placement or removal of shield 260, base 262 optionally includes a projecting flange 270, which may include recesses on its opposed sides to form tactile surfaces on the either side of flange 270 to further enhance a user's grip of and ability to place shield 260. Additionally, base 262 may include a visual indication 272, such as a triangle, which a user can use to align with the stop 212b, which may be in the form of an arrow, to assure proper alignment of the shield 260 on nozzle 212.

Referring now to FIG. 9, bone preparation system 210 includes the hand piece 211, nozzle 212, both described above, along with tube set 14 (described in reference to the first embodiment) and a shield 260 (optional), also described above. In addition to being enclosed in a package 215, similar to package 15 described above, the bone preparation system 210 includes a carrier 215a to hold the various components in a planar arrangement to improve the organization of the components and protect them during transport.

Carrier 215a may be formed from a sheet of plastic with a plurality of couplers arranged in a plane. Each coupler may be formed from cuts outs 215b in the sheet that can be folded over and interconnected to form loops 215c around the components. Other cutouts may simply fold upwards with slots 215d to allow a component, such as a shield, to be held by simply being inserted into the slot. In this manner, when removed from the pouch, the various components will remain supported on the carrier and, further, allow the components to be disconnected in a sequence that reduces the chance of contamination.

For example, the end of the tube set may first be removed and connected to the CO₂ supply, and then the tubing may be unwound, with the hand piece then removed followed by the shield, which can then be mounted to the nozzle.

Referring to FIGS. 14 and 14A, as noted above in reference to hand piece 11, nozzle 212 may be configured to form a snap fit coupling with hand piece body 211a at output port 218b. As best seen in FIG. 14A, nozzle 212 includes an enlarged base 280, which forms collar 230 and includes a tapered passageway 280a that is in fluid communication with the internal passageway 211c of hand piece 211. Base 280 includes a stepped profile with an annular rim or ring 282. Ring 282 forms a channel 284 for receiving an O-ring seal 284a between ring 282 and the end of nozzle 212 to seal the nozzle 212 in hand piece body 211a. Ring 282 provides the rotational connection for the nozzle 212 in hand piece body 211a, while still remained sealed in the hand piece body via the o-rig seal. The same or similar connection may be used in the previous embodiments.

As best seen in FIGS. 12 and 14A, ring 282 may have an angled outer surface 282a, which matches the internal profile of hand piece body 211a in passageway 211c, and which also may be received in a notch 211d formed in hand piece body 211a in passageway 211c to form the snap fit coupling with hand piece body 211a. The hand piece body 211a may be configured to form a spring or snap arm 211e (FIG. 12) adjacent notch 211d to further enhance the snap fit coupling of nozzle 212 and, further, to form a frangible part so that if nozzle 212 is pulled from hand piece 211 the arm will break and hand piece 211 will no longer be usable. Thus, nozzle 212 and hand piece 211 have an interfacing snap fit design that allows for the nozzle to be rotated while maintaining the O-ring seal and the physical connection between these pieces. This is further facilitated by snap arm 211e that snaps over the ramped surface 282a of ring 282.

The internal configuration of any of the disposable hand pieces described above may have several additional optional features, as described below and illustrated in FIG. 14. For example, referring to FIG. 14, as noted above, the trigger 228 is pivotally mounted at one end via a pivot member 228a, which pivotally mounts trigger 228 about axis 228b in hand piece body 211a. The other end of trigger 228 includes a tab 229, which extends through internal wall 211g into the hollow space 211b' of hand grip 211b via a slotted opening 211h in wall 211g and, as such, mounts trigger 228 in hand piece body 211a for movement between its fully compressed position (where the internal passageway 211c is fully open) and its fully uncompressed position (where the internal passageway 211c is fully closed by the valve stem described below). Further, tab 229 prevents the trigger from pivoting beyond its closed position (as shown in FIG 14). Pivot member 228a and tab 229 may be both formed with trigger when molding trigger 228.

As noted above, trigger 228 controls the flow of fluid through internal passageway 211c of hand piece body 211a. To that end, trigger 228 supports a valve stem 286, which is mounted in a passageway 211f along valve axis 286a, which intersects with passageway 211c. In this manner, as valve stem 286 moves along valve axis 286a in passageway 211f, the internal passageway 211c of hand piece 211 will open or close to allow fluid to flow though hand piece 211. The amount of flow will, therefore, be controlled and modulated by the position of the valve stem 286 in passageway 211f, as controlled by trigger 228.

As best seen in FIG. 14, trigger 228 includes a recess 228d to support the end of valve stem 286 as the valve stem is moved through passageway 211f by trigger 228. To urge the trigger 228 in its fully closed position, hand piece 211 includes a spring 290, which applies a spring force on trigger 228 through its full range of motion and keeps trigger 228 closed until user activation. Spring 290 is a coil spring mounted between a projection 228, such a cylindrical trunnion, formed on trigger 228, and a similar projection 211j, such as cylindrical trunnion, formed in hand piece body 211a. Further, in the illustrated embodiment spring 290 is not directly on the axis 286a of valve stem 286, and instead is offset from valve stem 286. Optionally, spring 290 is oriented with its compression axis 290a so that it is generally parallel to valve axis 286a of valve stem 286 and, therefore, allows for a more compact hand piece assembly since the spring and flow valve can occupy adjacent vertical space. Spring 290 may comprise a non-linear spring or a linear spring so that a uniform applied force on trigger 28 will produce a linear response in the movement of valve stem 286.

Optionally, as best seen in FIG. 14, the input port 218a of hand piece 218 may have a fitting with one or more O-ring seals and a cannulated barbed end to facilitate the connection of the tubing of tube set 14 to hand piece 211.

Similar to hand piece 11, hand piece 211 may also include a U-shaped channel 294 on the topside of hand piece 211 above grip 211b, which is configured to provide a frictional grip of additional tubing 16 or tube 16a inserted therein, which may be used, for example, to provide suction or which forms of a light pipe to project light on to the bone being prepared. Channel 294 may be formed when molding body 211a. Again, ribs and/or a resilient or elastomeric material may be molded on the inside of channel 294 to increase the friction forces on tubing inserted therein.

In any of the above embodiments (as well as variations thereof), the nozzle tips of the various hand pieces may be varied and, further, configured to clean or dry trunnions on implants. Many orthopedic implants utilize a trunnion to connect mating, modular components to them. A trunnion is a carefully engineered truncated cone feature on the implant that provides a place to mate the modular component, which has a matching conically tapered trunnion receptacle portion. The surfaces of these trunnion features must be precisely shaped and machined to very close tolerances to ensure a very precise fit so that micro motion between the two parts joined by the trunnion is minimized. These modular components in orthopedic implants typically experience very high cyclic loads, making the surfaces that are joined by the trunnion susceptible to fretting wear and other forms of corrosion. Wear and corrosion due to this micro motion has been shown to liberate metal debris that can be very damaging and toxic to both nearby and remote tissues in the patient. Therefore, in addition to having available carefully designed and machined trunnion components to implant, the surgeon must ensure that, during the surgical procedure, the trunnion surfaces are as clean as possible before assembly of the modular components so that their precise interfit is not compromised in any way. The implant that incorporates the male portion of the trunnion joint is typically implanted first and is left exposed to blood and other tissue debris during the procedure. The modular component that incorporates the female portion of the trunnion connection is typically opened from its package and immediately applied to the male portion so is therefore less exposed to contamination in the surgical field.

The present bone preparation systems would preferably be offered in several different configurations or 'kits', each intended for a specific application. For example, one kit might be intended for use during knee replacement surgery, and another for use during hip or shoulder replacement surgery. The nozzle designs are preferably optimized for each of these applications and would thus be different, resulting in different kit configurations. Accordingly, any of the above hand pieces may be fitted with a nozzle tip that is configured to prepare the male portion of a trunnion.

Referring to FIG. 16, in one example of a nozzle tip 354, nozzle tip 354 is configured with a cone-shaped body 300 that is suitable for use with cleaning trunnions. Cone shaped body 300 may be formed from a non-metallic material, such as plastic, so as to prevent possible scratching or damage of the trunnion during use. As best seen in FIG. 16, one end 354a of cone shaped body is tapered down and configured to form an inlet port 302 attached to or formed with the respective nozzle (e.g. in place of nozzle tips 54, 154, 254) of a hand piece. Compressed sterile CO₂ gas can, therefore, be introduced through inlet port 302 from the respective hand piece onto a trunnion 310 located in cone-shaped chamber or space 300c formed by cone-shaped body 300. As described more fully below, nozzle tip 354 is configured to direct the gas downwardly onto the trunnion 310.

In the illustrated example, cone-shaped body 300 includes an inner conical wall 300a and an outer conical wall 300b to define an inner space there between that forms into which gas flow is directed from inlet port 302. Body 300 may be molded as a one piece member from a single material or molded as a one piece from multiple materials, such as by co-injection molding, or may be assembled from multiple pieces (e.g. each molded) and joined together, for example by fasteners or welding. Therefore, at least the inner wall may be formed from a non-metallic material, such as plastic.

Inner conical wall 300a forms cone-shaped chamber or space 300c (with a central cone axis) that is sized to form fit over and receive trunnion 310 therein and, further, forms a space there between to allow the CO₂ gas to flow over the trunnion, and also allow the CO₂ gas stream to impact the trunnion surface at a desired angle. To that end, inner conical wall 300a includes a plurality of passageways 304 that form apertures or openings 304a, which are arranged optionally in a uniform spacing along wall 300a (both vertically and horizontally). Passageways 304 are optionally angled downwardly to form an acute angle relative to the central cone axis so that when CO₂ flows from the openings 304a, the gas will be directed downwardly onto the trunnion 310. The number and size and shaped of openings may vary. In the illustrated example, the passageways are be circular in cross-section in wall 300a but form oblique openings 304 at inner wall surface 300c. The shape of the openings 304 may be varied, however, as noted.

Openings 304a, therefore, can generate vectors 306 of compressed CO₂ being directed distally (towards the trunnion) to push fluid away or down along and away from the trunnion. Additional openings 308 may also be located in the base rim of cone-shaped body 300 in a base wall 300d, which connects the inner and outer walls 300a and 300b together. CO₂ flowing through these openings can clean an exposed bone surface. Cone-shaped body 300 may be designed to mate with, for example, a femoral stem trunnion 310, which should be kept clean to prevent mechanically assisted crevice corrosion (MACC) and subsequent adverse local tissue reaction (ALTR).

As shown in FIG. 17A, the numeral 454 designates yet another example, of a nozzle tip that is designed to direct the gas onto a trunnion 410. In the illustrated example, the nozzle tip is configured to direct the gas upwardly (and therefore forms an obtuse angle relative to the central axis of the cone). In this manner, the gas and liberated debris can flow up and into an outlet 422 provided in the form of a collection cup 422a in the upper portion of nozzle tip 454, which is then in fluid communication with a conduit 424, which directs the gas and debris to the outside of the nozzle tip for and includes a connection 424a for connection with a suction connection 424a, which in turn connects a suction tube.

In the illustrated example, similar to nozzle tip 354, nozzle tip 454 is formed from a conical-shaped body with an inner wall 400a and an outer wall 400b, which define there between a space into which the gas is flowed from inlet port 402 (which is coupled to a supply tube 402a that connects to a gas supply) and directed onto the trunnion 410 (positioned therein) positioned in chamber 400c via passageways 404. Similarly, body 400 may be formed from a non-metallic material, such as plastic, so as to prevent possible scratching or damage of the trunnion during use, and may be molded as a one piece member from a single material or molded as a one piece from multiple materials, such as by co-injection molding, or may be assembled from multiple pieces (e.g. each molded) and joined together, for example by fasteners or welding.

In this example, as noted, passageways 404 are angled upwardly so that opening 404a are also oblique but direct the flow of gas upwardly onto a trunnion positioned in nozzle tip 454.

For example, nozzle tip 454 may be configured to cover or mate with, for example, a femoral stem trunnion 410, which should be kept clean to prevent MACC and subsequent ALTR. An alternate configuration can place the suction connection at the top of the trunnion cleaner (offset from the compressed gas inlet) so that suction flow is in alignment with the direction the fluid and debris is being blown. With both versions, the gas could be directed via small holes (as depicted) or via a semi-circumferential angled slot at the base of the cone (not depicted).

Referring to FIG. 17B, the numeral 554 designates yet another example of a nozzle tip. In the illustrated embodiment, nozzle tip 554 is also formed from a conical-shaped body 500. Similarly, body 500 may be formed from a non-metallic material, such as plastic, so as to prevent possible scratching or damage of the trunnion during use, and may be molded as a one piece member from a single material or molded as a one piece from multiple materials, such as by co-injection molding, or may be assembled from multiple pieces (e.g. each molded) and joined together, for example by fasteners or welding.

Body 500 includes an inner wall 500a and an outer wall 500b, which define there between a space into which the gas is flowed from inlet port 502 (supply tube not shown) and directed onto the trunnion (positioned therein) positioned in chamber 500c via passageways 504. The gas and debris are then directed to an outlet 522, provided in the form of a collection cup 522a, similar to the previous example.

In this example the passageways 504 are arranged in a single row around and adjacent the base opening of body 500, to create an "air knife" (focused pressure gradient) that is directed upwards along the surface of the trunnion, to urge the fluidic debris upwards toward the vacuum collection point at outlet 522. Similar to passageways 404, passageways 504 are angled upwardly so that opening 504a are also oblique but direct the flow of gas upwardly. For further details of the collection cup and tubing that directs the gas and liberated debris away from the trunnion and to discharge tube or the like reference is made to the above example.

Referring to FIG. 17C, the numeral 654 designates yet another example of a nozzle tip. In the illustrated embodiment, nozzle tip 654 is also formed from a conical-shaped body 600 with an inner wall 600a and an outer wall 600b, which define there between a space into which the gas is flowed from inlet port 602 through an inlet tube 602a, which maybe coupled to a high pressure source. Similarly, body 600 may be formed from a non-metallic material, such as plastic, so as to prevent possible scratching or damage of the trunnion during use, and may be molded as a one piece member from a single material or molded as a one piece from multiple materials, such as by co-injection molding, or may be assembled from multiple pieces (e.g. each molded) and joined together, for example by fasteners or welding.

In the illustrated example, body 600 directs gas onto the trunnion (positioned in chamber 600c) via one or more annular passageways 604, which may also generate an "air knife".

As best seen in FIG. 17D, body 600 includes a central opening 600d, which is in fluid communication with the chamber 600c and which extends through the upper end or top of the body to thereby form an outlet 622 (see e.g. FIG. 17C). An inverted collection cup 622a may be located spaced from outlet 622 and includes a connection 622b for connection to a vacuum tube (not shown) to suction gas and debris that impinges on the collection surface 622c of cup 622a. The vacuum tube is coupled to a suction source so that the gas and debris may be removed, similar to the previous examples.

In the illustrated embodiment, as best seen in FIG. 17E, the passageway 604 comprises a single annular passageway or slit formed in inner wall 600a, optionally around and adjacent the base opening of body 600. Similar to passageways 504, passageway 604 may be angled upwardly so that opening 604a directs the flow of gas upwardly. Alternately, passageway 604 may be at least approximately perpendicular to the central axis of the cone.

Thus passageway 604 also creates an "air knife" (focused pressure gradient) that is directed upwards along the surface of the trunnion positioned in chamber 600c (even when perpendicular the air knife may be directed upwardly depending on the shape of the trunnion) to urge the gas and fluidic debris upwards toward the vacuum collection point at port 622. With the high pressure gas flowing into inlet 602, high pressure gas jets, labeled as arrows A (FIG. 17D) are generated-which flow into chamber 600c as well as toward outlet 622. The annular slit may also induce a secondary flow of ambient air, CO₂, and debris (arrows labeled B in FIG. 17D and 17E) in the chamber 600c entering at the base opening and then flowing towards the outlet 622 at the top of body 600. In this sense, the induced flow acts as a "compressed air" vacuum.

Optionally, as noted, collection cup 622a may be spaced from outlet 622 to form one or more vents so that the debris and gases exiting from the top of the device could pass towards low pressure atmosphere. With compressed air vacuums, the gas and debris flow typically go into a fabric bag that captures the debris and allows the gas to escape to atmosphere. In this manner, the gas flow changes direction and heavier substances fall out on the bend and collide with a collecting surface. In this example, the change of direction of the gas is the labeled with arrow C, and the heavier substances are designated using arrows D. Thus, the active suction line is attached to the collection cup 622a at the center of body 600 and includes a collecting surface 622c, which can straddle the opening at outlet 622 to capture and remove the collected fluid debris as it occurs.

Optionally, collection cup 622a may be attached to body 600, but, as noted, at a spacing or distance to thereby form one or more vents, which allows the overall gas flow to vent efficiently through a circumferential gap between the cup and body 600, and capturing the debris to be evacuated by the suction tube instead of requiring the use of sponges or towels. Alternatively, possibly a filter bag could be employed as previously mentioned.

While some of the nozzle tips for cleaning trunnions are shown absent the nozzles or hand pieces, it should be understood that they could also be included as part of the system, like the other nozzle tips shown herein. Further, while several of the nozzle tips are described in the context of use with trunnions, these nozzle tips may also be used on other tapered or cylindrical shaped object to be cleaned. Additionally, features described in reference to one tip may be incorporated into the other tips describe herein. Therefore, the use of the term "embodiment" is for convenience only and not intended to limit the scope of the claims.

Although not specifically mentioned for each of the above components, any of the hand pieces and their respective components parts (and accessories) may be made from plastic or metal or both. Though as noted, in some instances a plastic construction may be more suitable, for example, to reduce scratching or damage of the bone or the trunnion. Further, the hand pieces and their respective components parts (and accessories) may be molded, such as by injection molding, including co-injection molding, or cast molding or 3D printed or the like. They may be formed as a one-piece (unitary) construction or from multiple pieces that are then assembled together.

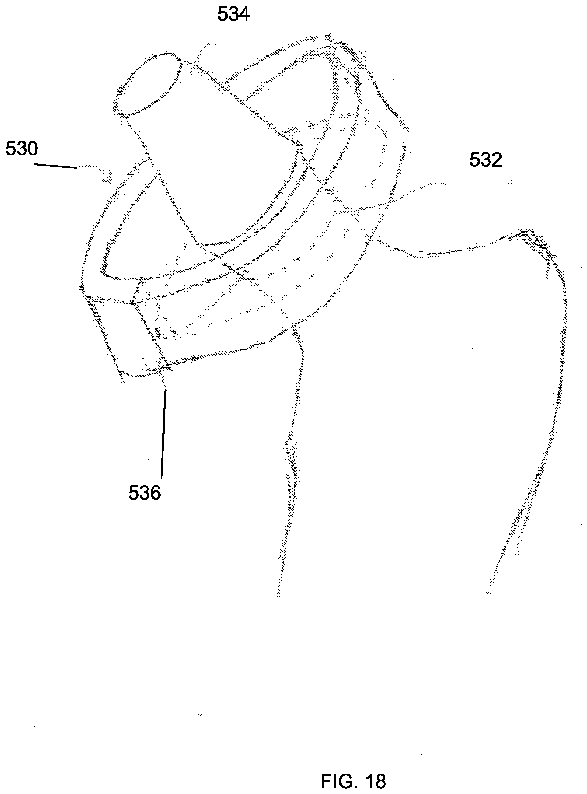

When using any of the nozzle tips, a disposable (surgical drape or similar) or reusable (silicone or similar) sleeve 530 may be used. Referring to FIG. 18, sleeve 530 may be configured as an annular ring that attaches to the nozzle tip, such as nozzle tips 354, 454, 554, or 654 and includes a central opening 532, for example, a slotted opening or an opening with a plurality of radially extending slots, so that it can be slid down the trunnion, e.g. trunnion 534, and adapted to the circumference of the trunnion or bone. Sleeve 530 may be used to catch and /or absorb a majority of displaced blood and tissue debris. This sleeve can be in place while the femoral or humeral head is assembled onto the trunnion, which is a feature that ensures that the trunnion can be kept clean through the assembly process. If necessary, the sleeve could be composed of an inner absorbable material and an outer impermeable layer. This sleeve could have a split 536 along at least one side to allow for easy removal (and assembly). This sleeve would be most advantageous for the trunnion cleaner nozzle tip example shown in FIG. 16, due to the direction of the gas flow which could otherwise direct blood and debris beyond the surgical working site.

A potential method or workflow would be: The trunnion is cleaned of gross debris (wiped or similar); the sleeve is inserted; the trunnion is given a final cleaning; the prosthetic head is mated to the trunnion; finally, the sleeve is removed.

While several forms of the hand piece, nozzle, tip, shield, and packaging have been shown and described, other forms will now be apparent to those skilled in the art. For example, one or more of the features of one hand piece may be incorporated into the other hand pieces descried herein. Similarly, one or more of the features of one tip may be incorporated into the other tips described herein. In addition, one or more of the features of one shield may be incorporated into the other shields described herein, and one or more of the features of one packaging or packaging arrangement may be incorporated into the other packaging or packaging arrangements described herein. As noted above, the use of the term "embodiment" is used for convenience to refer to the illustrated combinations and not intended to limit the scope of the invention, which is defined by the claims.

## Claims

1. A bone preparation system (210) comprising:
a hand piece (211) having an inlet port (218a) and an outlet port (218b), said hand piece having a hand piece body (211a) that forms said inlet port and said outlet port, said hand piece body further forming an internal passageway (211c) extending through said hand piece body from said inlet port to said outlet port to form a flow path from said inlet port to said outlet port, said hand piece having a valve stem (286) supported therein for movement across and at least partially within said internal passageway for opening and closing said internal passageway wherein flow along said flow path is blocked by said valve stem, and said hand piece having a trigger (228) configured to move said valve stem at least partially within said internal passageway to thereby control the flow of fluid through said hand piece;
a nozzle (212) coupled to said hand piece at said output port, said nozzle having a nozzle tip (254); and
tubing (16) coupled to said hand piece at said input port for coupling said bone preparation system to a supply of fluid, **characterised in that** said internal passageway has a longitudinal axis extending therethrough and defines a linear flow path extending along said longitudinal axis from said inlet port to said outlet port of said hand piece body.

2. The bone preparation system according to claim 1, wherein said trigger (228) is movable from a fully open position wherein said internal passageway (211c) is fully open and a fully closed position wherein said internal passageway is fully closed, further comprising a spring (290) to bias said trigger in its fully closed position.

3. The bone preparation system according to claim 2, wherein said valve stem (286) moves along a valve axis (286a), and said spring (290) applies a biasing force to said trigger (228) along an axis (290a) adjacent to said valve axis, and optionally wherein said spring applies a biasing force to said trigger along an axis parallel to said valve axis.

4. The bone preparation system according to any preceding claim, wherein said nozzle (212) is coupled to said hand piece (211) at said output port by a snap fit coupling.

5. The bone preparation system according to claim 4, wherein said hand piece body includes a spring arm (211e) adjacent said outlet port to form said snap fit coupling.

6. The bone preparation system according to any preceding claim, further comprising a splash shield (260) for mounting to said nozzle (212), said splash shield configured to form a snap fit mounting to said nozzle, and optionally wherein said nozzle includes guide structures to guide mounting said splash shield to said nozzle, and further optionally wherein one of said guide structures forms a stop (212b) to stop said splash shield from moving along said nozzle once mounted to said nozzle.

7. A bone preparation system kit comprising:
a bone preparation system (210) according to any preceding claim, said hand piece (211), said nozzle (212) and said tubing (16) being sterilized wherein said bone preparation system is sterilized; and
a package (15) enclosing said bone preparation system, wherein said package is configured to maintain the sterilization of said bone preparation system.

8. The bone preparation system kit according to claim 7, wherein said package (15) is formed from a material that allows sterilization of said bone preparation system while enclosed as assembled in said package, and optionally further comprising a carrier (215a) to support said bone preparation system in said package, and further optionally, wherein said carrier supports said bone preparation system in a planar arrangement.

9. The bone preparation system kit according to claim 7, wherein said package (15) comprises a pouch.

10. The bone preparation system kit according to claim 8, wherein said carrier (215a) includes a plurality of couplers arranged in a planar array to couple each of said nozzle (212), said hand piece (211) and said tubing (16) to said carrier.

11. The bone preparation system kit according to claim 10, further comprising a splash shield (260) for mounting to the nozzle (212), the shield being supported by said carrier (215a) and enclosed in said package (15).

## Patentansprüche

1. Knochenpräparationssystem (210), umfassend:
ein Handstück (211) mit einer Einlassöffnung (218a) und einer Auslassöffnung (218b), wobei das Handstück einen Handstückkörper (211a) aufweist, der die Einlassöffnung und die Auslassöffnung bildet, wobei der Handstückkörper ferner einen internen Durchgang (211c) bildet, der sich durch den Handstückkörper von der Einlassöffnung zu der Auslassöffnung erstreckt, um einen Strömungsweg von der Einlassöffnung zu der Auslassöffnung zu bilden, wobei das Handstück einen Ventilschaft (286) aufweist, der darin zur Bewegung über und zumindest teilweise innerhalb des internen Durchgangs zum Öffnen und Schließen des internen Durchgangs gestützt ist, wobei die Strömung entlang des Strömungswegs durch den Ventilschaft blockiert wird, und wobei das Handstück einen Auslöser (228) aufweist, der dazu konfiguriert ist, den Ventilschaft zumindest teilweise innerhalb des internen Durchgangs zu bewegen, um dadurch die Fluidströmung durch das Handstück zu steuern;
eine Düse (212), die an der Ausgangsöffnung an das Handstück gekoppelt ist, wobei die Düse eine Düsenspitze (254) aufweist; und
Schlauchmaterial (16), das an der Eingangsöffnung an das Handstück gekoppelt ist, um das Knochenpräparationssystem an eine Fluidversorgung zu koppeln, **dadurch gekennzeichnet, dass** der interne Durchgang eine Längsachse aufweist, die sich dadurch erstreckt und einen linearen Strömungsweg definiert, der sich entlang der Längsachse von der Einlassöffnung zu der Auslassöffnung des Handstückkörpers erstreckt.

2. Knochenpräparationssystem nach Anspruch 1, wobei der Auslöser (228) aus einer vollständig offenen Position, wobei der interne Durchgang (211c) vollständig offen ist, und einer vollständig geschlossenen Position, wobei der interne Durchgang vollständig geschlossen ist, bewegbar ist, ferner umfassend eine Feder (290), um den Auslöser in seiner vollständig geschlossenen Position vorzuspannen.

3. Knochenpräparationssystem nach Anspruch 2, wobei sich der Ventilschaft (286) entlang einer Ventilachse (286a) bewegt und die Feder (290) eine Vorspannkraft auf den Auslöser (228) entlang einer Achse (290a) benachbart zu der Ventilachse ausübt, und optional wobei die Feder eine Vorspannkraft auf den Auslöser entlang einer Achse parallel zu der Ventilachse ausübt.

4. Knochenpräparationssystem nach einem vorhergehenden Anspruch, wobei die Düse (212) an der Ausgangsöffnung durch eine Schnappverbindung an das Handstück (211) gekoppelt ist.

5. Knochenpräparationssystem nach Anspruch 4, wobei der Handstückkörper einen Federarm (211e) benachbart zu der Auslassöffnung beinhaltet, um die Schnappverbindung zu bilden.

6. Knochenpräparationssystem nach einem vorhergehenden Anspruch, ferner umfassend ein Spritzschild (260) zum Montieren an der Düse (212), wobei das Spritzschild dazu konfiguriert ist, eine Schnappmontage an der Düse zu bilden, und optional wobei die Düse Führungsstrukturen beinhaltet, um das Montieren des Spritzschildes an der Düse zu führen, und ferner optional wobei eine der Führungsstrukturen einen Anschlag (212b) bildet, um zu verhindern, dass sich das Spritzschild entlang der Düse bewegt, sobald es an der Düse montiert ist.

7. Knochenpräparationssystem-Kit, umfassend:
ein Knochenpräparationssystem (210) nach einem vorhergehenden Anspruch, wobei das Handstück (211), die Düse (212) und das Schlauchmaterial (16) sterilisiert sind, wobei das Knochenpräparationssystem sterilisiert ist; und
eine Verpackung (15), die das Knochenpräparationssystem umschließt, wobei die Verpackung dazu konfiguriert ist, die Sterilisation des Knochenpräparationssystems aufrechtzuerhalten.

8. Knochenpräparationssystem-Kit nach Anspruch 7, wobei die Verpackung (15) aus einem Material gebildet ist, das eine Sterilisation des Knochenpräparationssystems ermöglicht, während es im zusammengebauten Zustand in der Verpackung umschlossen ist, und optional ferner umfassend einen Träger (215a), um das Knochenpräparationssystem in der Verpackung zu stützen, und ferner optional, wobei der Träger das Knochenpräparationssystem in einer planaren Anordnung stützt.

9. Knochenpräparationssystem-Kit nach Anspruch 7, wobei die Verpackung (15) einen Beutel umfasst.

10. Knochenpräparationssystem-Kit nach Anspruch 8, wobei der Träger (215a) eine Vielzahl von Kopplern beinhaltet, die in einem planaren Array angeordnet sind, um jedes von der Düse (212), dem Handstück (211) und dem Schlauchmaterial (16) an den Träger zu koppeln.

11. Knochenpräparationssystem-Kit nach Anspruch 10, ferner umfassend ein Spritzschild (260) zum Montieren an der Düse (212), wobei das Schild durch den Träger (215a) gestützt und in der Verpackung (15) umschlossen ist.

## Revendications

1. Système de préparation osseuse (210) comprenant :
une pièce à main (211) comportant un orifice d'entrée (218a) et un orifice de sortie (218b), ladite pièce à main comportant un corps de pièce à main (211a) qui forme ledit orifice d'entrée et ledit orifice de sortie, ledit corps de pièce à main formant en outre un passage interne (211c) s'étendant à travers ledit corps de pièce à main dudit orifice d'entrée audit orifice de sortie pour former un trajet d'écoulement dudit orifice d'entrée audit orifice de sortie, ladite pièce à main comportant une tige de valve (286) supportée à l'intérieur de celle-ci pour se déplacer à travers et au moins partiellement à l'intérieur dudit passage interne pour ouvrir et fermer ledit passage interne, dans lequel l'écoulement le long dudit trajet d'écoulement est bloqué par ladite tige de valve, et ladite pièce à main comportant une gâchette (228) configurée pour déplacer ladite tige de valve au moins partiellement à l'intérieur dudit passage interne pour ainsi commander l'écoulement de fluide à travers ladite pièce à main ;
une buse (212) couplée à ladite pièce à main au niveau dudit orifice de sortie, ladite buse comportant une pointe de buse (254) ; et
un tube (16) couplé à ladite pièce à main au niveau dudit orifice d'entrée pour coupler ledit système de préparation osseuse à une alimentation en fluide, **caractérisé en ce que** ledit passage interne comporte un axe longitudinal s'étendant à travers celui-ci et définit un trajet d'écoulement linéaire s'étendant le long dudit axe longitudinal depuis ledit orifice d'entrée jusqu'audit orifice de sortie dudit corps de pièce à main.

2. Système de préparation osseuse selon la revendication 1, dans lequel ladite gâchette (228) est mobile entre une position entièrement ouverte dans laquelle ledit passage interne (211c) est entièrement ouvert et une position entièrement fermée dans laquelle ledit passage interne est entièrement fermé, comprenant en outre un ressort (290) pour solliciter ladite gâchette dans sa position entièrement fermée.

3. Système de préparation osseuse selon la revendication 2, dans lequel ladite tige de valve (286) se déplace le long d'un axe de valve (286a), et ledit ressort (290) applique une force de sollicitation à ladite gâchette (228) le long d'un axe (290a) adjacent audit axe de valve, et facultativement dans lequel ledit ressort applique une force de sollicitation à ladite gâchette le long d'un axe parallèle audit axe de valve.

4. Système de préparation osseuse selon l'une quelconque des revendications précédentes, dans lequel ladite buse (212) est couplée à ladite pièce à main (211) au niveau dudit orifice de sortie par un raccord à encliquetage.

5. Système de préparation osseuse selon la revendication 4, dans lequel ledit corps de pièce à main comprend un bras de ressort (211e) adjacent audit orifice de sortie pour former ledit raccord par encliquetage.

6. Système de préparation osseuse selon l'une quelconque des revendications précédentes, comprenant en outre une protection anti-éclaboussures (260) destinée à être montée sur ladite buse (212), ladite protection anti-éclaboussures étant configurée pour former un montage à encliquetage sur ladite buse, et facultativement dans lequel ladite buse comprend des structures de guidage pour guider le montage de ladite protection anti-éclaboussures sur ladite buse, et en outre facultativement dans lequel l'une desdites structures de guidage forme une butée (212b) pour empêcher ladite protection anti-éclaboussures de se déplacer le long de ladite buse une fois montée sur ladite buse.

7. Kit de système de préparation osseuse comprenant :
un système de préparation osseuse (210) selon l'une quelconque des revendications précédentes, ladite pièce à main (211), ladite buse (212) et ledit tube (16) étant stérilisés dans lequel ledit système de préparation osseuse est stérilisé ; et
un emballage (15) enveloppant ledit système de préparation osseuse, dans lequel ledit emballage est conçu pour maintenir la stérilisation dudit système de préparation osseuse.

8. Kit de système de préparation osseuse selon la revendication 7, dans lequel ledit emballage (15) est formé à partir d'un matériau qui permet la stérilisation dudit système de préparation osseuse lorsqu'il est enveloppé tel qu'assemblé dans ledit emballage, et comprenant en outre facultativement un support (215a) pour supporter ledit système de préparation osseuse dans ledit emballage, et en outre facultativement, dans lequel ledit support supporte ledit système de préparation osseuse dans un agencement planaire.

9. Kit de système de préparation osseuse selon la revendication 7, dans lequel ledit emballage (15) comprend une poche.

10. Kit de système de préparation osseuse selon la revendication 8, dans lequel ledit support (215a) comprend une pluralité de raccords agencés en un réseau plan pour coupler chacun de ladite buse (212), de ladite pièce à main (211) et dudit tube (16) audit support.

11. Kit de système de préparation osseuse selon la revendication 10, comprenant en outre une protection anti-éclaboussures (260) destinée à être montée sur la buse (212), la protection étant supportée par ledit support (215a) et enveloppée dans ledit emballage (15).
